# NEUE EUROPÄISCHE PATENTSCHRIFT

(11) **EP 0 776 652 B2**
(45) Veröffentlichungstag und Bekanntmachung des Hinweises auf die Entscheidung über den Einspruch: **18.10.2000**
(45) Hinweis auf die Patenterteilung: 17.12.1997
(21) Anmeldenummer: 96117796.1
(22) Anmeldetag: 07.11.1996
(51) Int. Cl.: A61K 7/13

(54) **Oxidations-Haarfärbemittel enthaltend mindestens einen Pflanzenextrakt und mindestens ein Eiweisshydrolysat**
Oxidation hair dyes containing at least one plant extract and at least one protein hydrolyzate
Colorants d'oxydation des cheveux contenant au moins un extrait de plantes et au moins un hydrolysat de proteine

(30) Priorität: 30.11.1995 DE 19544655
(43) Veröffentlichungstag der Anmeldung: 04.06.1997
(73) Patentinhaber: GOLDWELL GmbH, D-64297 Darmstadt (DE)
(72) Erfinder: Lorenz, Heribert, 64401 Gross-Bieberau (DE); Misu, Daisuke, 64289 Darmstadt (DE); Wilz, Rüdiger, 64297 Darmstadt (DE)

(56) Entgegenhaltungen:
- EP-A- 0 186 025
- EP-A- 0 642 783
- DE-A- 3 320 539
- DE-A- 3 609 962
- FR-A- 2 694 018
- US-A- 4 839 168
- DATABASE WPI Week 9150 Derwent Publications Ltd., London, GB; AN 91-365788 XP002027614 "Dyeing agent for hair - comprises diamine oxidn. dye, vegetable extract contg. pyrogallol tannin and zeolite carrying transition metal" & JP 03 246 217 A (NOEVIER) , 1.November 1991
- DATABASE WPI Week 9108 Derwent Publications Ltd., London, GB; AN 91-56427 XP002027702 T. ROSLYAKOV ET AL : "Hair dyeing compsn. - contains additional carbonate extracts of carrot and camomile seeds, to improve properties" & SU 1 553 128 A (KRASD FOOD & NEVINNOMYSSK CHEM) , 30.März 1990
- DATABASE WPI Week 9140 Derwent Publications Ltd., London, GB; AN 91-291712 XP002027615 "Hair-dyeing agent compsn. , dyeing grey hair easily and gradually - comprises a ferrous salt(s) and propyl gallate, or an ascorbic cpd. and/or plant extracts and a nonionic surfactant(s)" & JP 03 193 722 A (LION) , 23.August 1991
- JP 5-221838 referiert als Online-Referat 119:256286 bei Chemical Abstract

## Beschreibung

Die vorliegende Erfindung betrifft ein Haarfärbemittel, das mindestens eine Entwickler- und mindestens eine Kupplersubstanz enthält, das unter Einfluß eines Oxidationsmittels auf dem Haar eine brillante, glänzende Färbung ergibt, haarschonend ist und auch noch eine gute Konditionierwirkung aufweist.

Oxidationsfärbemittel zur permanenten Haarfärbung sind seit Jahrzehnten bekannt. Ihre Wirkungsweise besteht darin, eine Mischung aus Farbstoffvorprodukten, sogenannten Entwickler- und Kupplersubstanzen, in einem geeigneten Trägermaterial mit einem Oxidationsmittel auf menschliches Haar aufzubringen, wobei sich durch Reaktion der genannten Materialien dann auf dem Haar die erwünschte Färbung entwickelt. Anschließend wird das Haar gewaschen und getrocknet.

Die bekannten Haarfärbemittel können dabei die verschiedensten Zusatzstoffe enthalten, es wird hierzu beispielsweise auf die Monographie von K. Schrader, "Grundlagen und Rezepturen der Kosmetika", 2. Aufl. (1989, Hüthig Buch Verlag), S. 784 bis 815, verwiesen.

Trotz aller Vielfalt der aus dem Stand der Technik bekannten Haarfärbemittel besteht noch immer ein Bedürfnis nach Verbesserungen, insbesondere der Brillanz und des Glanzes der erzielten Haarfärbungen bei gleichzeitiger Schonung des Haares und gegebenenfalls konditionierender Wirkung.

Es wurde nunmehr gefunden, daß ein Haarfärbemittel mit entsprechenden Eigenschaften erhalten wird, wenn man einem Entwicklersubstanz(en) und Kuppler-Substanz(en) in üblicher Grundlage enthaltendem, mit Oxidationsmitteln reagierenden Gemisch mindestens einen Pflanzenextrakt und mindestens ein pflauzliches Eiweißhydrolysat, vorzugsweise in einer Menge von 0,1 bis 3,0 Gew.-% bzw. 0,1 bis 2,5 Gew.-%, insbesondere 0,3 bis 1,2 Gew.-%, bezogen auf die Gesamtzusammensetzung, zusetzt.

Besonders geeignet sind dabei pflanzliche Eiweißhydrolysate wie solche von Glykoproteinen mit einem hohen Anteil an Hydroxyprolin-Gruppen, wie sie insbesondere durch Hydrolyse von Pflanzenzellwandbestandteilen erhalten werden und die vorzugsweise auch Serin enthalten. Ebenso geeignet sind Pflanzenprotein-Hydrolysate, ausgehend von Sojaprotein, Weizenprotein, Maisprotein, Erdnußprotein, sowie Erbsen, Reis, Korn, Kartoffeln, Bohnen, Cashew, Walnüssen, Erdnüssen und auch Mandelprotein (vgl. EP-A 186 025).

Das Molgewicht dieser Pflanzenprotein-Hydrolysate liegt in der Regel im Bereich zwischen etwa 500 und etwa 100 000, typischerweise zwischen 800 und etwa 50 000, insbesondere zwischen etwa 1 000 und 30 000, besonders bevorzugt zwischen etwa 1 000 und 5 000. Sie werden durch enzymatische oder Säure-Hydrolyse nach an sich bekannten Verfahren erhalten.

Bei den erfindungsgemäß verwendeten Pflanzenextrakten handelt es sich vorzugsweise um alkoholische, wäßrigalkoholische und wäßrige (z. B. Wasserdampf-) Zubereitungen von Pflanzenteilen wie Blättern, Früchten, Blüten, Wurzeln, Rinden oder Stämmen.

Geeignete Pflanzenextrakte sind z. B. solche aus Aloe, Arnika, Baldrian, Bilsenkraut, Birke, Brennesseln, Echinacea, Efeu, Enzian, Farnen, Fichtennadeln, Ginster, Hafer, Hamamelis, Holunder, Hopfen, Huflattich, Kamillen, Kastanien, Klee, Lindenblüten, Maiglöckchen, Melisse, Mistel, Passionsblumen, Ratanhia, Ringelblumen, Rosmarin, Roßkastanien, Rotdorn, Salbei, Schachtelhalm, Schafgarbe, Schlüsselblumen, Taubnesseln, Thymian, Weinblättern, Weißdorn, etc.

Geeignete Handelsprodukte sind beispielsweise die verschiedenen "Extrapone®", "Sedaplant" und "Hexaplant". Geeignete Extrakte und deren Herstellung sind auch in "Hagers Handbuch der pharmazeutischen Praxis", 4. Aufl., beschrieben.

Der Anteil an Extrakten in den Haarfärbemitteln ist natürlich abhängig von deren Wirkstoff-Konzentration und liegt zwischen etwa 0,005 bis etwa 2,5 Gew.-%, vorzugsweise etwa 0,05 bis etwa 1,5 Gew.-%, besonders bevorzugt bei etwa 0,1 bis etwa 1 Gew.-%, berechnet auf den Aktivgehalt an Wirkstoff bzw. Trockenrückstand und die Gesamtzusammensetzung des Mittels.

Geeignete Entwicklersubstanzen sind vor allem die bekannten Phenylendiamine und deren Derivate, z. B. 1,4-Diaminobenzol und 2,5-Diaminotoluol, 4-Aminophenol, 1-Hydroxyethyl-2,5-diaminobenzol (vgl. EP-A 7537 und EP-B 400 330), Tetraaminopyrimidine, Triaminohydroxypyrimidine, insbesondere 2,5,6-Triamino-4-hydroxypyrimidin (vgl. EP-A 467 026), 5-Aminosalicylsäure (vgl. EP-A 345 728) und 2-(2'-Hydroxyethyl-)amino-5-aminotoluol (vgl. EP-A 615 743).

In Abhängigkeit vom gewünschten Farbton sind im Prinzip alle bekannten und zugelassenen Kupplersubstanzen zum Einsatz in den erfindungsgemäßen Haarfärbemitteln geeignet.

Beispielhaft seien insbesondere Resorcin, 2-Methylresorcin, 4-Chlorresorcin, 2-Aminophenol, 4-(N-Methyl)aminophenol, 3-Aminophenol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodiphenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol, und/oder 1-Methoxy-2-amino-4-(2'-hydroxyethylamino)benzol genannt.

Der Anteil an Entwicklersubstanz liegt üblicherweise bei etwa 0,05 bis etwa 5, vorzugsweise 0,1 bis 3, insbesondere 0,25 bis 2 Gew.-%, berechnet auf die Gesamtzusammensetzung des Mittels, und, falls die Entwicklersubstanz in Form eines Salzes vorliegt, berechnet auf die freie Base.

Das molare Verhältnis von Entwicklersubstanz(en) zu Kupplersubstanz(en) liegt üblicherweise bei etwa 1 : 1 bis etwa 2,5 : 1.

Es können selbstverständlich auch, zur Erzielung besonderer Farbtöne, Gemische aus verschiedenen Kupplersubstanzen eingesetzt werden, insbesondere solche aus Resorcin oder 2-Methylresorcin mit 2-Aminophenol und/oder 3-Aminophenol, aus Resorcin oder 2-Methylresorcin und 1-Methoxy-2-amino-4-(2'-hydroxyethylamino)benzol, Gemische aus 2-Amino-3-hydroxypyridin mit 5-Amino-2-methylphenol und/oder 1-Methoxy-2-amino-4-(2'-hydroxyethylamino)benzol, oder auch 1,3-Diaminobenzol mit 1,4-Diamino-2-chlorbenzol.

Falls erwünscht, können zur Erzielung bestimmter Farbnuancen auch übliche direktziehende Farbstoffe, beispielsweise die bekannten Arianor-Farbstoffe oder auch Nitrofarbstoffe wie 2-Amino-4,6-dinitrophenol, 2-Amino-6-chlor-4-nitrophenol, 2-Amino-4-nitrophenol etc., sowie auch bekannte Pflanzenfarbstoffe, beispielsweise Henna, in geringen Mengen, d.h. zwischen 0,05 und 1 Gew.-%, enthalten sein. Die Gesamtmenge des Farbstoffgemisches im Endprodukt beträgt vorzugsweise etwa 0,2 bis etwa 6,0 Gew.-%, insbesondere etwa 0,5 bis etwa 4 Gew.-%, des Haarfärbemittels.

Zur Herstellung des erfindungsgemäßen Haarfärbemittels werden die Oxdationsfarbstoff-Vorprodukte, d.h. das Gemisch aus Entwicklersubstanz und Kupplersubstanz und ggf. anwesenden direktziehenden Farbstoffen, in einen geeigneten kosmetischen Träger eingearbeitet. Solche sind insbesondere Emulsionen, d.h. Cremes, oder Gele.

Solche Zusammensetzungen und die in ihnen enthaltenen weiteren Stoffe, insbesondere oberflächenaktive Substanzen, Stabilisatoren, Verdickungsmittel etc. sind einschlägiger Stand der Technik und in verschiedenen Monographien, beispielsweise bei K.Schrader, "Grundlagen und Rezepturen der Kosmetika", 2.Aufl. (1989, Hüthig Buchverlag), S. 796 bis 815, beschrieben. Auf diese und andere Monographien wird daher ausdrücklich Bezug genommen.

Die erfindungsgemäßen Zusammensetzungen werden unmittelbar vor der Anwendung mit peroxidhaltigen Zusammensetzungen, beispielsweise mit jeweils gleichen Anteilen 6prozentiger Wasserstoffperoxid-Lösung, vermischt und auf menschliches Haar aufgebracht, wo sie nach etwa 15- bis 30-minütiger Einwirkung mit Wasser und einem üblichen Shampoo wieder ausgewaschen werden.

Die an sich bevorzugt verwendeten Wasserstoffperoxid-Zusammensetzungen können durch andere Peroxid-Zubereitungen ersetzt sein, beispielsweise Perborate, Harnstoffperoxid, Melaminperoxid, etc.; jedoch gestaltet sich die Dosierung und Handhabung solcher Zubereitungen, die bis zur Anwendung wasserfrei gehalten werden müssen, umständlicher.

Im folgenden wird die Erfindung anhand von Ausführungsbeispielen illustriert.

### I.

In ein Trägermaterial der Zusammensetzung

| | |
|---|---|
| Laureth-2 | 20,0 (Gew.-%) |
| Nonoxynol-4 | 2,5 |
| Ölsäure | 15,0 |
| Isopropylalkohol | 14,0 |
| 1,2-Propandiol | 0,5 |
| Natriumsulfit | 1,0 |
| EDTA, Tetranatriumsalz | 0,2 |
| Ammoniumchlorid | 0,5 |
| Ammoniak | 2,0 |
| Wasser | @ 100,0 |

wurden, unter entsprechender Verringerung des Wassergehaltes, folgende Zusammensetzungen eingearbeitet:

### Beispiele

| (Angaben in Gew.-%) | | | | |
|---|---|---|---|---|
| | **1** | | **2** | |
| | **A** | **B** | **A** | **B** |
| Hydrolysiertes Weizenprotein | - | 0,55 | 0,50 | 0,50 |
| Hopfenextrakt | - | 0,50 | - | - |
| Roßkastanienextrakt | - | 0,50 | - | - |
| Kamillenextrakt | - | - | - | 0,50 |
| Weißdornextrakt | - | - | - | 0,50 |
| 1,4-Diaminobenzol | 0,30 | 0,30 | 0,20 | 0,20 |
| Resorcin | 0,15 | 0,15 | - | - |
| 3-Aminophenol | 0,15 | 0,15 | 0,03 | 0,03 |
| 4-Aminophenol | - | - | 0,60 | 0,60 |
| 4-Amino-2-hydroxytoluol | 0,05 | 0,05 | 0,20 | 0,20 |
| 1-Naphthol | - | - | 0,20 | 0,20 |
| Natriumpikramat | 0,05 | 0,05 | - | - |

Die Zusammensetzungen wurden jeweils mit 6%-iger wäßriger Wasserstoffperoxid-Lösung vermischt, wobei ein pH-Wert von 9,6 erhalten wurde.

Die Mischungen wurden jeweils halbseitig auf das Haar aufgebracht (auf eine Hälfte Zusammensetzung A, auf die andere Hälfte Zusammensetzung B), nach 30-minütiger Einwirkung wurde shampooniert und Glanz und Brillanz der Färbungen miteinander verglichen.

### Ergebnis:

Die mit den Zusammensetzungen 1 A und 1 B behandelten Haarhälften wiesen eine mahagonirote Färbung auf, wobei die mit der Zusammensetzung 1 B gefärbte Hälfte einen deutlich verbesserten Farbglanz gegenüber der mit der Zusammensetzung 1A gefärbten Hälfte zeigte; auch Halt, Griff, Weichheit und Volumen des Haares waren deutlich verbessert.

Die mit der Zusammensetzung 2 gefärbten Haare wiesen einen kupferbrillanten Farbton auf; die mit der Zusammensetzung 2B behandelte Haarhälfte war gegenüber derjenigen mit 2A behandelten hinsichtlich Farbglanz und Volumen eindeutig überlegen.

### II.

In ein Trägermaterial der Zusammensetzung

| | |
|---|---|
| Cetylstearylalkohol | 11,0 (Gew.-%) |
| Natriumcetylstearylsulfat | 1,5 |
| Oleth-5 | 5,0 |
| Stearamide MEA | 2,0 |
| Cocamide MEA | 2,0 |
| Ölsäure | 3,0 |
| Propylenglykolmono/distearat | 0,5 |
| EDTA, Tetranatriumsulfat | 0,2 |
| Natriumsulfit | 1,0 |
| Ammoniak | 2,1 |
| Wasser | @ 100,0 |

wurden, unter entsprechender Verringerung des Wassergehalts, folgende Bestandteile eingearbeitet:

### Beispiele

| (Angaben in Gew.-%) | | | | |
|---|---|---|---|---|
| | **3** | | **4** | |
| | **A** | **B** | **A** | **B** |
| Hydrolysiertes Weizenprotein | - | 0,50 | - | 0,50 |
| Hopfenextrakt | - | 0,50 | - | - |
| Roßkastanienextrakt | - | 0,50 | - | - |
| Kamillenextrakt | - | - | - | 0,50 |
| Salbeiextrakt | - | - | - | 0,50 |
| 2,5-Diaminotoluolsulfat | 0,50 | 0,50 | 1,00 | 1,00 |
| Resorcin | 0,20 | 0,20 | - | - |
| 2-Aminophenol | 0,05 | 0,05 | - | - |
| 4-Aminophenol | - | - | 0,10 | 0,10 |
| 4-Amino-2-hydroxytoluol | - | - | 0,40 | 0,40 |

Die Anwendung und Entwicklung erfolgte wie unter I. beschrieben; der pH-Wert der gebrauchsfertigen Zusammensetzungen lag bei 9.7.

### Ergebnis:

Die mit den Zusammensetzungen 3A und 3B gefärbten Haarhälften zeigten eine mittelblonde Farbe, während mit den Zusammensetzungen 4A und B eine mahagoniviolette Färbung erzielt wurde.

Die mit den Zusammensetzungen 3B und 4B behandelten Haarhälften wiesen eine stärkere Farbbrillanz, höheren Glanz und darüber hinaus einen besseren Griff, Volumen und Fülle gegenüber den mit den Zusammensetzungen 3A und 4A behandelten Haarhälften auf.

### III.

In ein Trägermaterial der Zusammensetzung

| | |
|---|---|
| Cetylstearylalkohol | 9,0 (Gew.-%) |
| Natriumcetylstearylsulfat | 1,0 |
| Stearamide MEA | 1,6 |
| Cocoamide MEA | 1,6 |
| Ölsäure | 1,0 |
| PEG-5-Cocoamide | 0,6 |
| EDTA, Tetranatriumsalz | 0,2 |
| Natriumsulfit | 0,3 |
| Ammoniumchlorid | 0,5 |
| Mangandioxid | 0,1 |
| Wasser | @ 100,0 |

wurden, unter entsprechender Verringerung des Wassergehalts, folgende Bestandteile eingearbeitet:

### Beispiele

| (Angaben in Gew.-%) | | | | |
|---|---|---|---|---|
| | **5** | | **6** | |
| | **A** | **B** | **A** | **B** |
| Sojaproteinhydrolysat | - | 0,60 | - | 0,35 |
| Hopfenextrakt | - | 0,50 | - | - |
| Salbeiextrakt | - | - | - | 1,00 |
| Echinacea-Extrakt | - | 0,50 | - | - |
| 2,5,6-Triamino-4-hydroxypyrimidinsulfat | 0,48 | 0,48 | 0,75 | 0,75 |
| 2,5-Diaminopyridin | 0,25 | 0,25 | 0,40 | 0,40 |
| 2,6-Diaminopyridin | 0,01 | 0,01 | 0,03 | 0,03 |
| 2-Methylresorcin | 0,10 | 0,10 | - | - |
| 4-Chlorresorcin | - | - | 0,10 | 0,10 |
| HC-Red 3 | 0,15 | 0,15 | - | - |
| 3-Amino-2-methylamino-6-methoxypyridin | 0,03 | 0,03 | 0,15 | 0,15 |
| Natriumpicramat | - | - | 0,10 | 0,10 |

Die Haarfärbung erfolgte durch Vermischen der Zusammensetzungen 5A, 5B, 6A und 6B mit 2%-iger Wasserstoffperoxid-Lösung im Verhältnis 1 : 2 (pH-Wert: 6,8), 20-minütiger Einwirkung auf das Haar, Shampoonieren und Trocknen.

Mit den Zusammensetzungen 5A und 5B wurde eine rotbraune, mit den Zusammensetzungen 6A und 6B eine kastanienbraune Färbung erhalten.

Im Verhältnis zu den mit den Zusammensetzungen 5A und 6A gefärbten Haarhälften wiesen die mit den Zusammensetzungen 5B und 6B gefärbten Haarhälften einen deutlich ausgeprägteren Glanz und ein verbessertes Volumen mit vollem Griff auf.

## Patentansprüche

1. Oxidations-Haarfärbemittel auf Basis eines Entwickler-Kuppler-Systems in einem Trägermaterial, enthaltend mindestens einen Pflanzenextrakt und zusätzlich mindestens ein pflanzliches Eiweißhydrolysat.

2. Haarfärbemittel nach Anspruch 1, enthaltend 0,1 bis 2,5 Gew.-%, bezogen auf die Gesamtzusammensetzung, mindestens eines pflanzlichen Eiweißhydrolysats.

## Claims

1. Oxidative hair dyeing composition on the basis of a developer-coupler system in a suitable carrier, comprising at least one plant extract and, in addition, at least one vegetable protein hydrolyzate.

2. Hair dyeing composition according to claim 1, comprising 0.1 to 2,5 % by wt., calculated to the total composition, of at least one vegetable protein hydrolyzate.

## Revendications

1. Composition de teinture d'oxydation des cheveux à base d'un système révélateur-coupleur dans une composition de support, contenant d'au moins un extrait végétal et en plus d'au moins un hydrolysat de protéines végétales.

2. Composition de teinture des cheveux sélon la revendication 1, contenant entre 0.1 et 2.5% en poids, calculés à la composition totale, d'un hydrolysat de protéines végétales.
